# EUROPEAN PATENT SPECIFICATION

(11) **EP 0 763 344 B1**
(45) Date of publication and mention of the grant of the patent: **21.11.2001**
(21) Application number: 96306626.1
(22) Date of filing: 12.09.1996
(51) Int. Cl.: A61B 8/00, A61B 8/08

(54) **Portable ultrasonic diagnostic apparatus**
Tragbares Ultraschall-Diagnostikgerät
Appareil diagnostique portatif à ultrasons

(30) Priority: 13.09.1995 KR 9529912
(43) Date of publication of application: 19.03.1997
(73) Proprietor: MEDISON CO., LTD., Kangwon-do 250-870 (KR)
(72) Inventor: Chang, Seon Ho, Kangnam-gu, Seoul (KR)
(74) Representative: Sturt, Clifford Mark

(56) References cited:
- EP-A- 0 219 988
- NL-A- 8 800 016
- US-A- 3 964 296
- US-A- 4 393 712
- US-A- 5 295 485
- US-A- 5 361 767

## Description

The present invention relates to a portable ultrasonic diagnostic apparatus, and more particularly, to a pistol-shaped portable ultrasonic diagnostic apparatus.

An ultrasonic diagnostic apparatus scans an ultrasonic wave into a human body and converts information loaded on a reflected ultrasonic wave into an electrical signal using a piezoelectric effect of a probe, to thereby display the electrical signal on a screen. Therefore, the ultrasonic diagnostic apparatus can enable a user to obtain information of the inside of a human body, without cutting out the affected parts of the human body.

However, a conventional ultrasonic diagnostic apparatus is bulky and weighty, which should be improved. Even in a compact model, it weighs more than 10Kg, which makes it difficult to move it and impossible to carry it.

NL 8800016 A discloses a hand held ultrasonic diagnostic apparatus having a pivotable video screen and two ultrasonic probes. Various control buttons are provided on the housing of the apparatus to facilitate a user to operate it.

US 3964296 also discloses an ultrasonic diagnostic apparatus. The apparatus may be in the shape of a pistol and have an on/off trigger and a probe drum, which is provided in the muzzle of the gun.

To solve the above problem, it is an object of the present invention to provide a portable ultrasonic diagnostic apparatus.

To accomplish the above object of the present invention, there is provided a portable ultrasonic diagnostic apparatus comprising:
a case;
a probe which is formed in said case;
an electronic circuit, which is installed in said case, for converting a frequency signal supplied from said probe into a video signal to be displayed;
a display, which is installed in a portion opposite to said probe on said case, for displaying the video signal supplied from said electronic circuit thereon; and
a user manipulation keypad which is installed in a predetermined portion to facilitate a user to operate the ultrasonic diagnostic apparatus or to select various functions of the ultrasonic diagnostic apparatus, characterised by further comprising:
   a lamp for facilitating diagnosis in a dark place,
   wherein said case is a pistol-shaped case and said probe is formed in a muzzle of said pistol-shaped case.

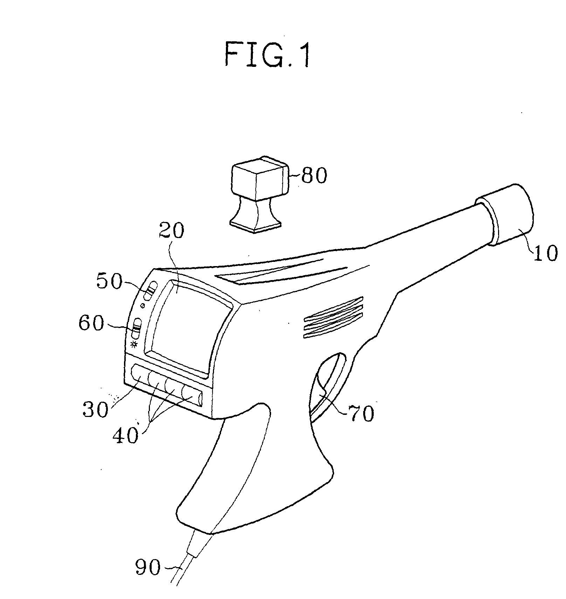

FIG. 1 is a schematic view of a portable ultrasonic apparatus according to the present invention.

A preferred embodiment of the present invention will be described below in more detail, by way of example only, with reference to the accompanying diagrammatic drawing.

A probe 10 scans an ultrasonic wave and converts a reflected ultrasonic wave into an electrical signal. It is preferable that a single element probe is used as the probe to minimise the volume and weight of the ultrasonic diagnostic apparatus.

An electronic circuit for processing an analog signal and a digital signal to perform ultrasonic diagnosis is installed in the body of the pistol-shaped ultrasonic diagnostic apparatus. A display 20 displays an ultrasonic diagnosis result of which the signal has been processed, on a screen. It is preferable that such a display is a LCD display. Since the LCD display has a small and thin screen, it is appropriate for a portable ultrasonic diagnostic apparatus. A user can adjust the brightness of the screen on which the image is displayed, using a brightness control key 50. Also, the user can adjust the contrast of shape of the displayed image using a contrast control key 60.

Power for driving the ultrasonic diagnostic apparatus is supplied via a power cable 90 or using a battery such as a dry cell. The user drives the portable ultrasonic diagnostic apparatus by depressing a power key 30. Function select keys 40 enable the user to select various functions of the ultrasonic diagnostic apparatus. The portable ultrasonic diagnostic apparatus according to the present invention can possess a picture stop function as an auxiliary function. That is, using a freeze key 70, a particularly displayed picture can be stopped during the ultrasonic diagnosis, or the stopped picture can be changed into a picture which is being actually scanned. Also, the portable ultrasonic diagnostic apparatus according to the present invention includes a detachable lamp 80 to enable the user to facilitate diagnosis in a dark place.

As described above, the portable ultrasonic diagnostic apparatus according to the present invention may include an LCD display for displaying the result of the ultrasonic diagnosis and a single element probe as a probe in a pistol-shaped case, thereby minimising the volume and the weight of the ultrasonic diagnostic apparatus and allowing more convenient use.

The aforegoing description has been given by way of example only and it will be appreciated by a person skilled in the art that modifications can be made without departing from the scope of the present invention.

## Claims

1. A portable ultrasonic diagnostic apparatus comprising:
a case;
a probe (10) which is formed in said case;
an electronic circuit, which is installed in said case, for converting a frequency signal supplied from said probe into a video signal to be displayed;
a display (20), which is installed in a portion opposite to said probe on said case, for displaying the video signal supplied from said electronic circuit thereon; and
a user manipulation keypad (30, 40, 50, 60) which is installed in a predetermined portion to facilitate a user to operate the ultrasonic diagnostic apparatus or to select various functions of the ultrasonic diagnostic apparatus, **characterised by** further comprising:
a lamp (80) for facilitating diagnosis in a dark place,
wherein said case is a pistol-shaped case and said probe is formed in a muzzle of said pistol-shaped case.

2. A portable ultrasonic diagnostic apparatus according to claim 1, wherein said probe is a single element probe.

3. A portable ultrasonic diagnostic apparatus according to claim 1 or claim 2, wherein said display is a liquid crystal display.

4. A portable ultrasonic diagnostic apparatus according to any preceding claim, further comprising a freeze key (70), located in a position where a convenient use of the user is enabled, for selecting a function of stopping a particularly displayed picture and changing the stopped picture into an actually scanned picture.

5. A portable ultrasonic diagnostic apparatus according to any one of the preceding claims, wherein said lamp is detachable from said pistol-shaped case.

## Patentansprüche

1. Tragbare Ultraschall-Diagnosevorrichtung, die umfaßt:
ein Gehäuse;
einen Prüfkopf (10), der im Gehäuse ausgebildet ist;
eine elektronische Schaltung, die im Gehäuse installiert ist, um ein vom Prüfkopf geliefertes Frequenzsignal in ein anzuzeigendes Videosignal umzusetzen;
eine Anzeigevorrichtung (20), die an einem Abschnitt gegenüberliegend dem Prüfkopf am Gehäuse installiert ist, um das von der elektronischen Schaltung gelieferte Videosignal anzuzeigen; und
ein Benutzermanipulation-Tastenfeld (30, 40, 50, 60), das in einem vorgegebenen Abschnitt installiert ist, um einem Benutzer die Bedienung der Ultraschall-Diagnosevorrichtung oder die Auswahl verschiedener Funktionen der Ultraschall-Diagnosevorrichtung zu erleichtern; **dadurch gekennzeichnet, daß** sie femer umfaßt:
eine Lampe (80) zur Erleichterung der Diagnose an einem dunklen Ort; wobei
das Gehäuse ein pistolenförmiges Gehäuse ist und der Prüfkopf in einer Rohrmündung des pistolenförmigen Gehäuses ausgebildet ist.

2. Tragbare Ultraschall-Diagnosevorrichtung nach Anspruch 1, bei der der Prüfkopf ein Einzelelementprüfkopf ist.

3. Tragbare Ultraschall-Diagnosevorrichtung nach Anspruch 1 oder Anspruch 2, bei der die Anzeigevorrichtung eine Flüssigkristallanzeigevorrichtung ist.

4. Tragbare Ultraschall-Diagnosevorrichtung nach irgendeinem der vorangehenden Ansprüche, die ferner eine Einfriertaste (70) umfaßt, die an einer Position angeordnet ist, an der eine bequeme Verwendung durch den Benutzer ermöglicht wird, um eine Funktion zum Festhalten eines besonderen angezeigten Bildes und zum Wechseln vom festgehaltenen Bild zu einem derzeit abgetasteten Bild auszuwählen.

5. Tragbare Ultraschall-Diagnosevorrichtung nach irgendeinem der vorangehenden Ansprüche, bei der die Lampe vom pistolenförmigen Gehäuse abnehmbar ist.

## Revendications

1. Appareil de diagnostic aux ultrasons portable comprenant :
un boîtier ;
une sonde (10) qui est formée dans ledit boîtier ;
un circuit électronique, qui est installé dans ledit boîtier, pour convertir un signal de fréquence délivré depuis ladite sonde en un signal vidéo à afficher ;
un affichage (20) qui est installé dans une partie opposée à ladite sonde sur ledit boîtier, pour afficher le signal vidéo délivré depuis ledit circuit électronique sur celui-ci ; et
un pavé de touches de manipulation par l'utilisateur (30, 40, 50, 60) qui est installé dans une partie prédéterminée pour faciliter l'opération de l'appareil de diagnostic aux ultrasons ou la sélection des diverses fonctions de l'appareil de diagnostic aux ultrasons par l'opérateur, **caractérisé en ce qu'**il comprend, en outre :
une lampe (80) pour faciliter les diagnostics dans un endroit sombre,
dans lequel ledit boîtier est un boîtier en forme de pistolet et ladite sonde est formée dans une bouche du boîtier en forme de pistolet.

2. Appareil de diagnostic aux ultrasons portable selon la revendication 1, dans lequel ladite sonde est une sonde à seul élément.

3. Appareil de diagnostic aux ultrasons portable selon la revendication 1 ou la revendication 2, dans lequel ledit affichage est un affichage à cristaux liquides.

4. Appareil de diagnostic aux ultrasons portable selon l'une quelconque des revendications précédentes, comprenant en outre une touche de figeage (70) placée à une position où elle permet une utilisation commode de l'utilisateur pour sélectionner une fonction d'arrêt d'une image affichée particulièrement et pour changer l'image arrêtée en une image actuellement balayée.

5. Appareil de diagnostic aux ultrasons portable selon l'une quelconque des revendications précédentes, dans lequel ladite lampe est détachable dudit boîtier en forme de pistolet.
